# EUROPEAN PATENT APPLICATION

(11) **EP 2 548 604 A1**
(43) Date of publication of application: **23.01.2013**
(21) Application number: 12170339.1
(22) Date of filing: 22.01.2002
(51) Int. Cl.: A61M 25/01, A61M 25/09

(54) **Non-metallic guide wire**

(30) Priority: 26.01.2001 US 770342
(62) Divisional of application: 07023141.0
(71) Applicant: Lake Region Manufacturing, Inc., Chaska, MN 55318 (US)
(72) Inventor: Fleischhacker, Mark G., Minnetonka, MN Minnesota 55345 (US)
(74) Representative: Parry, Simon James

(57) **Abstract**

A guide wire having a non-metallic, non-woven core wire is disclosed. Monofilar, polymeric fibers of multifilar helically-wound non-metallic fibers are preferred core wire materials. The guide wire optionally includes further coatings and other materials on the core wire. In one embodiment, a non-metallic distal coil wire is disclosed. The guide wire of this invention is particularly useable for magnetic resonance imaging applications.

## Description

### CROSS-REFERENCE TO RELATED APPLICATIONS

Not Applicable

### BACKGROUND OF THE INVENTION

Guide wires are used in various medical procedures to gain vascular or non-vascular access to anatomical locations. The guide wire is initially introduced into the anatomy of a patient by means of a needle or other access device, which in many procedures pierces the patient's skin. The guide wire is then advanced to a chosen or targeted anatomical location to provide a means of tracking guidance and support for other diagnostic, interventional, or therapeutic medical devices having lumens which can follow or track over a guide wire. Once such other medical devices reach their desired anatomical location, the guide wire is or can be withdrawn. The physician then proceeds with the protocol of the procedure. A specific but non-limiting example of the above is the placement of a balloon catheter at the site of a vascular blockage. Suffice it to say, guide wires are one of the most commonly used medical devices where vascular or arterial access is desired.

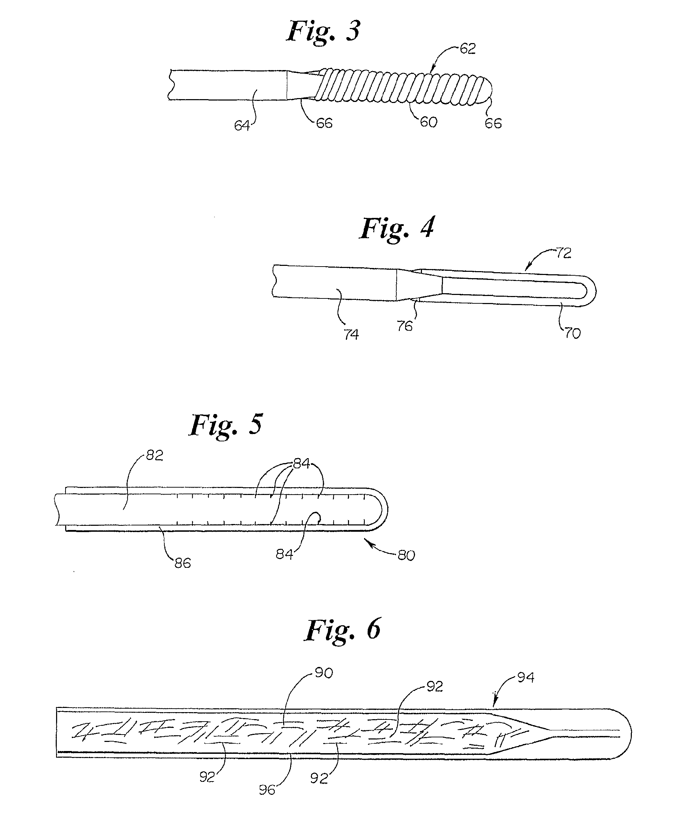

United States patent 5,705,014 to Schenck et al. discloses and claims methods for constructing instruments, specifically medical instruments, intended for use during a magnetic resonance (MR) imaging procedure. Essentially, the Schenck *et al.* '014 patent discloses methods for selecting carbon fiber/substrate composite materials and for doping such composites with materials of differing degrees of magnetization. In accordance with the teaching of Schenck *et al.,* the composite materials are doped so that medical instruments manufactured from the doped composites do not interrupt the MR imaging process or distort an image developed therefrom. The entirety of the disclosure of the Schenck *et al.* U.S. '014 patent is incorporated by reference herein. United States patent 5,251,640 to Thomas A. Osbourne discloses a "Composite Wire Guide Shaft". The '640 patent discloses a composite guide shaft comprising a multifilar core (See FIGS. 3, 4, and 5) having multiple fibers wrapped therearound, the entire structure being held together by, for example, an adhesive matrix. In one embodiment of the device described in the '640 patent, a hollow core wire guide is contemplated. Metal core wires are also discussed. The disclosure of the '640 patent is also incorporated by reference herein.

### BRIEF SUMMARY OF THE INVENTION

Briefly, in one aspect, the present invention is an elongate guide wire comprising a guide wire body or core wire, the body having distal, medial, and proximal segments or portions. The guide wire body of the present invention is substantially non-metallic, non-woven, and non-braided. In a preferred practice a guide wire core wire of this invention is polymeric. In a preferred practice, a guide wire body of this invention is monofilament and is substantially solid in cross-section throughout substantially its entire length.

A guide wire of this invention optionally may include a non-metallic coil wire. Guide wires of this invention are particularly useable during MR diagnostic and therapeutic procedures. In addition a guide wire of the present invention is kink resistant having the ability to prolapse, *i.e.,* to be bent backward, without kinking. A guide wire of this invention also is pushable, steerable, and torque transmissive, primarily from its proximal end. These terms will be more extensively defined below.

In a further embodiment of the present invention, the guide wire may comprise a non-metallic, helically-wound monofilar or multifilar core wire, or guide wire body embedded in a matrix material to provide a substantially solid (in cross-section) structure. A solid core wire structure of this aspect of the present invention may further comprise a coating such as is more completely described below.

In an alternative embodiment, a helical core wire guide wire may comprise one or more helical non-metallic coil wires wound about the core wire. The helically-wound coil wires may be held in place by means of an adhesive. The coil wire may be located adjacent any or all of the proximal, medial and distal segments of the guide wire. Usually the coil wire is axially or radially disposed around the distal segment. The helically-wound coil wires of this further aspect of the present invention may be wound in the same or opposite directions. One skilled in the art will appreciate that the selection of fiber composition and direction(s) of wind will significantly include the torque transmissive characteristics of the guide wire.

The term "guide wire" as used herein is to be broadly construed to mean essentially any wire-like structure of dimension and length which is intended to assist in the placement of a catheter or other medical device at a site of medical interest. Percutaneous procedures in which placement of a catheter or other device through the skin and into the vasculature, are a preferred category of medical procedures in which guide wires are used. Guide wire herein is intended to include but is not limited to what is usually referred to as a guide wire, a main wire, introducer guide wires, diagnostic, therapeutic or interventional guide wires, wire guides, and spring guide wires, but also includes exchange guide wires and extension wires. Dimensions of guide wires to which the present invention primarily applies fall in the range of about 0.012 in. to about 0.065 in. in diameter and about 30 cm to about 300 cm (or more) in length. Without limiting the generality of the foregoing, peripheral, cerebral (including neuro-interventional), guide wires or wire guides are within the contemplation of this definition. Guide wires of the present invention may include structure (*e.g*., on their extreme proximal segment) which permits them to be extended during a procedure by connection in a second (extension wire) guide wire. Guide wires of this invention also will generally have a reduced diameter, increased flexibility tip. Guide wires of this invention optionally may be coated or treated with various further compositions, *e.g.*, polymers or other compounds, to change their handling or performance characteristics such as to increase lubricity, to increase or decrease hydrophobicity,or to reduce thrombogenicity of their external surface. Guide wires of the invention may also be uncoated.

A guide wire of the present invention is said to be "non-metallic". This term is intended to mean containing or comprising no metals, alloys, or other materials which respond in some manner to the magnetic or radio frequency fields generated in an MR imaging system. This definition is intended to exclude any non-ferrous metals which, while not necessarily interacting with the MR magnetic fields, exhibit what has become known as "antenna effect" by interaction with the radio frequency fields used in that procedure. Thus magnetic field deflection and "antenna effect" are completely eliminated by the use of the present invention.

A preferred class of materials, which is non-metallic in accordance with this invention, comprises polymeric materials. Polymeric materials useable in the present invention are preferably hydrocarbon-based comprised of the elements of carbon and hydrogen. However, hydrocarbon polymer is useable in the present invention can, and often will, include oxygen, nitrogen, or other elements, usually as minor constituents.

### BRIEF DESCRIPTION OF THE FIGURES

The present invention will now be discussed in detail, the understanding of which will be enhanced by reference to the attached figures in which:
FIG. 1 is a cross-sectional view (partially broken away) of one embodiment of the present invention:
FIG. 2 is a second embodiment of the present invention in which a polymeric core and polymeric guide wire coil are used.
FIG. 3 is a further embodiment of the present invention in which a polymeric coil is disposed on the distal end of the guide wire core wire.
FIG. 4 is a cross sectional view of another embodiment of the present invention in which a polymeric jacket material is disposed on the distal end of the guide wire core wire.
FIG. 5 is a cross sectional view of a further embodiment of the present invention in which a substantially uniform diameter polymeric guide wire core which has been partially radially cut or scored to increase distal segment or distal tip flexibility.
FIG. 6 illustrates a guide wire core structure of the present invention comprising a polymeric core material in which there is disposed glass fiber segments and an optional external coating.

### DETAILED DESCRIPTION OF THE INVENTION

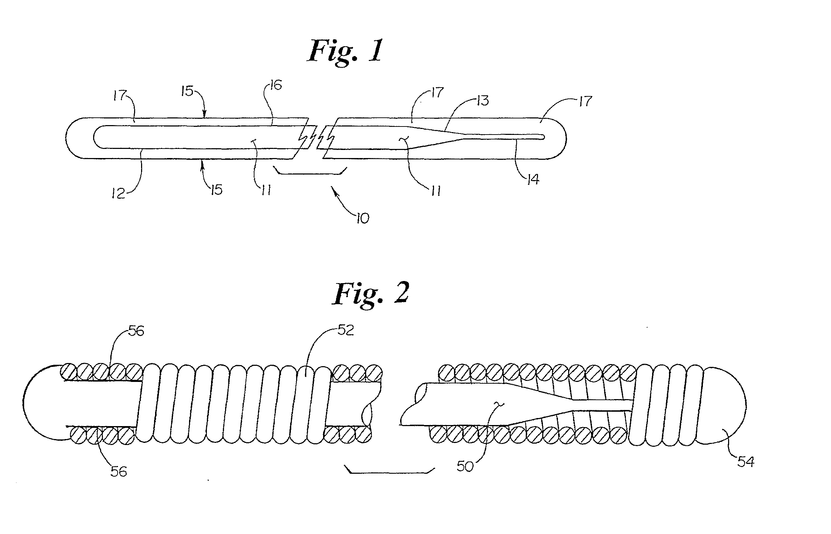

The invention will now be described with reference to the FIGs. noted above and the attached claims. FIG. 1 shows a partially broken away, cross sectional view of one embodiment of the present invention. FIG. 1 shows a guide wire 10 comprising a guide wire body or core wire 11 having a proximal segment 12, distal segment 14, and a medial segment 16. It is to be understood that the medial segment will generally comprise the majority of the length of the guide wire 10 and has been broken as shown for purposes of illustrating other features of the invention. The terminology of proximal, medial, and distal, as it is used with reference to guide wire structures, will be well understood by one skilled in this art to mean structures of the guide wire as determined from the user's perspective. More specifically, the distal segment 14 of a wire of this invention generally means that portion of the guide wire which first enters the patient's anatomy when the device is utilized. The distal segment 14 of any particular guide wire is generally designed to be more flexible than the rest of the guide wire. In that regard, the distal segment 14 begins with a taper 13 in which the medial segment 16 of the guide wire body has a gradually reduced diameter. Taper 13 leads to distal segment 14, which, as shown in this embodiment has a lesser diameter than medial segment 16, or proximal segment. Thus, distal segment 14 will generally be more flexible than medial segment 16. The diameter of distal segment 14 may be reduced, for example, by centerless grinding

The embodiment shown in the FIG. 1 includes an optional outer covering, coating, or jacket 17. Generally speaking jacket 17 will be a non-metallic polymeric material, the polymer of coating 17 being different from that of guide wire body 11. For example, one preferred polymer of coating 17 is PEBAX polyetherimide. Polyurethane, nylon, and polytetrafluoroethylene (PTFE) are further examples of optional coatings which could be used with the present invention. Extruded polymer coatings or other heat-shrunk polymer coatings also may be utilized. A variety of other hydrophilic, hydrophobic or other coatings that are known to one skilled in this art can optionally be used with the present invention. As is shown in FIG. 1, coating or jacket 17 tends to make the overall diameter (arrows 15) of the guide wire more uniform. Polymer coatings contemplated by the present invention optionally may include radiopaque fillers such as barium salts in order to enhance the visibility of the guide wire when used with non-resonance imaging systems.

Guide wire body 11 is non-metallic, and in a preferred practice, polymeric. The overall diameter of the guide wire of at least the medial segment shown in FIG. 1 (at arrows 15) is approximately 0.035 inches. A preferred polymeric material for guide wire body 11 polyetheretherketone, sold under the designation PEEK. PEEK as is used in accordance with this invention is commercially available from many sources. A preferred source is Zeus Industrial Products, Inc. in Orangeburg, South Carolina, U.S.A. (HTTP://www.zeusinc.com). PEEK is preferred for use in the present invention because it is camber resistant, having little tendency to break when sharply bent. It is also thermally stable permitting other polymeric materials to be extruded over it without change in dimension. PEEK is also believed to be capable of being impregnated with glass fibers, *e.g.*, to alter its handling characteristics. "Camber resistant" herein means having the property or tendency not to be become curved when held in a circular package while being shipped. Camber resistance could also be described as not having the tendency to remain curved or circular even though guide wires are commercially shipped in circular carriers. The absence of camber means that medical personnel using a device of this invention can remove it from its generally circular shipping tube (the device may have been maintained in a circular configuration for several months while the device was in inventory and being shipped) and still be immediately useable, *e.g.*, for catheter placement.

Polyetheretherketone described above also has the property of not being easily broken when sharply bent, *e.g.*, around human or other vasculature. PEEK also tends to allow prolapsing without kinking or fracturing. This is also an advantage of the use of PEEK to make the guide wire body of this invention. Last, as is noted above, the distal segment of a guide wire of the present invention is generally more flexible than either of the proximal or medial segments. In this instance, the polymer used should preferably be capable of being centerless ground. Being capable of being centerless ground means that the reduced diameter distal segment (14 in the FIG. 1) can easily be manufactured using conventional guide wire processing techniques.

A second material from which guide wire body 11 can comprise is a carbon fiber commercially available from SGL Carbon Corp. of Charlotte, N.C., U.S.A. The SGL Carbon fiber generally comprises bundled, helically-wound or twisted carbon fibers held together by means of an adhesive or other resin. A vinylester resin is a preferred adhesive or binder, the binder being applied by pultrusion of the wound carbon fibers or fiber bundles through a die. In a preferred practice of the present invention, the helically-wound guide wire body has at least 10 helical turns per foot. Helically-wound glass fibers, with an appropriate binder or adhesive, are believed to be similarly useable. Nylon fibers, and "Isoplast" glass filled plastic fibers commercially available from Dow Chemical Corporation, are also believed to be useable in this structure. A structure so constructed can be centerless ground, *e.g.*, on the distal portion thereof, so as to reduce its diameter and increase its flexibility.

The ability to control the flexibility of the distal portion of a guide wire of the present invention using well-known centerless grinding processes is one of the surprising and unexpected advantages of the present invention. Centerless grinding is a technique that is conventionally used to fabricate metallic guide wires. For example, centerless grinding is often used to reduce the diameter of a portion of a metal guide wire (*e.g.*, the distal portion of a guide wire core wire), to increase distal tip flexibility. Centerless grinding was a technique that, prior to this invention, was not believed to be useable for non-metallic guide wires. Centerless grinding of a portion of the guide wire body is much easier to accomplish than the use of staggered length, parallel, longitudinal fibers as is described in the above-mentioned Osboume U.S. '640 patent at col. 3 line 20.

The polymeric materials which have been found to be useful for fabricating the guide wire core wire or guide wire body have properties which are representative of the properties of any polymeric material from which a guide wire of this invention is to be fabricated. Specifically, the polymeric material must have sufficiently longitudinal rigidity or stiffness so that the guide wire can be advanced within a patient's vasculature in much the same fashion as *e.g.*, a conventional 0.035 in. (diameter) metal angiography wire. As is noted above, the material must also be camber resistant while also being resistant to prolapsing. Last, a workable polymeric material must be capable of being fabricated to have properties and "feel" like conventional metal, *e.g.*, medical grade stainless steel, guide wires. In summary, polymeric materials from which the instant guide wire body or core wire can be fabricated are those that, with similar diameters, lengths, and coatings tend to perform in a medical procedure substantially the same as their metallic counterparts.

It is to be noted that guide wire body 11 is substantially solid in section, substantially throughout its entire length. No interior lumens, or other void spaces are contemplated to be needed or necessary to practice the present invention presuming a polymeric material having the above characteristics is selected to fabricate the guide wire body.

FIG. 2 illustrates a second embodiment of the present invention wherein the guide wire comprises a solid guide wire core wire or body 50 comprises a polymeric material as disclosed herein with a polymeric coil wire 52 substantially disposed therearound. Core wire 50 and coil 52 may be attached to each other by any means suitable for adhering one polymeric material to another. For example, an adhesive may be used (at 54 and 56) to bond the guide wire components to each other. It is to be noted that coil wire 52 is wound around substantially the entire length of core wire 50 in this embodiment of the invention.

FIG. 3 is a further embodiment of the present invention in which a polymeric coil wire 60 is disposed on just the distal segment 62 of core wire 64. Polymeric or plastic coil wire materials include PEI (polyetherimide commercially available from General Electric Plastics and sold under the trade designation "Ultem"), PES (polyether sulfone commercially available from BASF under the trade designation "Ultrason") and various other high performance polymeric materials the identities of which would occur to one skilled in this art in view of this disclosure. Polymeric core 64 may comprise PEEK or carbon fiber as is described above. Adhesive joints 66 bind the coil sire to the core wire.

FIG. 4 illustrates a variation of the structure shown in FIG. 3 in which a polymer-based jacket material 70 is disposed on the distal segment 72 of guide wire core wire 74. An optional adhesive 76 may be used to adhere jacket material 70 to core wire 74. Illustrative polymeric jacket materials include, polyurethane and Pebax as is described above.

FIG. 5 illustrates a further embodiment of the present invention in which the distal segment 80 of polymeric guide wire core wire 82 has been made more flexible by cutting or etching therein a series of radial cuts 84. As will be understood (and as is illustrated), the depth and distance between cuts 84 may be adjusted to increase or decrease the flexibility of distal segment 80. The width of the cuts 84 also may be increased or decreased to change device tip flexibility. Also as is shown, an optional polymer-based coating 86 is disposed over distal segment 80. Polymer coating 86 may be disposed over all or part of core wire 82 as is well known in the art.

FIG. 6 illustrates a further embodiment of the present invention in which a polymer guide wire core 90 has randomly disposed therein fibrous segments or fibers 92 of a second polymeric material. For example, a PEEK core material having therein randomly distributed glass fiber segments may be employed. Guide wire distal segment 94 may have a reduced diameter as is shown. The guide wire core optionally may include a polymeric outer coating 96. Coating 96 may be hydrophilic, hydrophobic, or have other desirable characteristics.

It will be appreciated that guide wires of the present invention can be used in situations where no magnetic resonance imaging is intended. The materials of the present invention are considerably less expensive than conventional materials of guide wires for similar applications. For example, guide wires of the present invention could be used to replace stainless steel diagnostic and angiography guide wires. Guide wires of the present invention would be especially applicable for those procedures where no steerability is needed. Monofilament PIC wires, conventionally made of metal, also could be replaced by the present invention. Many of the above non-MR imaging applications, where metal (including shape memory alloys) are used could be accomplished using the present invention.

The above description and examples are intended to be illustrative and not limiting of the present invention. One skilled in the art will appreciate that there may be many variations and alternatives suggested by the above invention. These variations and alternatives are intended to be within the scope of this invention as set forth in the following claims.

Features of preferred embodiments of the invention are defined in the numbered clauses below:
A. A guide wire comprising a core wire having distal, medial and proximal segments, the core wire substantially comprising a non-metallic, non-woven, material.
B. A guide wire according to clause A wherein the core wire distal segment has a diameter which is less than that of the core wire medial and proximal segments.
C. A guide wire according to clause A wherein the diameters of the core wire distal, medial, and proximal segments are all substantially the same.
D. A guide wire according to clause A wherein the core wire has a polymeric coating thereon which covers substantially the entire length of the guide wire.
E. A guide wire according to clause A wherein core wire has a tapered segment between the medial segment and the distal segment.
F. A guide wire according to clause A wherein the core wire further comprises a taper which couples the medial segment and the distal segment and wherein substantially the entire core wire is covered with a polymeric material.
G. A guide wire according to clause A wherein the core wire comprises a polymeric material.
H. A guide wire according to clause A wherein the core wire comprises a polymeric material and the core wire is substantially completely covered with a second polymeric material.
I. A guide wire according to clause A wherein the distal segment of the core wire has a diameter which is less than that of the medial segment.
J. A guide wire comprising a core wire, the core wire having coupled proximal, medial, and distal segments, the core wire substantially completely comprising a polymeric material.
K. A guide wire according to clause J wherein the core wire is coated with a second polymeric material.
L. A guide wire according to clause J wherein the core wire comprises carbon fiber.
M. A guide wire according to clause J wherein the core wire comprises polyetheretherketone.
N. A guide wire according to clause J wherein the core wire is coated with PEBAX polyetherimide.
O. A guide wire according to clause J wherein the core wire comprises polyetheretherketone, and the core wire is coated with polyetherimide.
P. A guide wire according to clause O wherein the core wire distal segment is more flexible than either of the medial segment or the proximal segment.
Q. A guide wire according to clause O wherein the core wire distal segment is coupled to the core wire medial segment through a tapered segment and the distal segment has a diameter which is less than that of the medial segment.
R. A guide wire according to clause O wherein the polyetherimide coating has a hydrophilic coating thereover.
S. A guide wire comprising a core wire having coupled distal, medial, and proximal segments, the core wire comprising multiple, helically-wound, non-metallic fibers and a binder resin, the binder resin being uniformly dispersed between the fibers so as to fill any void space therebetween.
T. A guide wire according to clause S which further comprises a coil wire disposed about the distal segment.
U. A guide wire according to clause S where the non-metallic fibers comprise carbon and the binder resin comprises a vinyl ester.
V. A guide wire according to clause S wherein the helically-wound fibers are wound to no more than 10 helices per foot of guide wire length.
W. A guide wire comprising a core wire having coupled, distal, medial and proximal segments, the core wire comprising a single helically-wound non-metallic fiber and a binder resin, the binder resin being uniformly dispersed between the helices of the fiber so as to fill any void space therebetween and to provide steerability and torqueability to the guide wire.
X. A guide wire according to clause W which further includes a coil wire disposed about the distal segment.
Y. A guide wire comprising a core wire, the core wire having coupled distal, medial and proximal segments, the core wire substantially comprising a non-metallic, non-woven, polymeric material, the guide wire **characterized in that** the polymeric material is non-oriented.

## Claims

1. A guide wire comprising a core wire having a coupled distal (14), medial (16) and proximal (12) segments, the core wire comprising randomly dispersed non-metallic fibers or fiber segments (92) and a binder resin, the binder resin (90) being uniformly dispersed between the fibers so as to fill any void space therebetween.

2. A guide wire according to claim 1 which further comprises a coil wire disposed about the distal segment.

3. A guide wire according to claim 1 where the non-metallic fibers (92) comprise carbon and the binder resin (90) comprises a vinyl ester.

4. A guide wire according to claim 1 wherein the helically-wound fibers are wound to no more than 10 helices per foot of guide wire length.

5. A guide wire comprising a core wire having coupled, distal (14), medial (16) and proximal (12) segments, the core wire comprising a single helically-wound non-metallic fiber and a binder resin, the binder resin being uniformly dispersed between the helices of the fiber so as to fill any void space therebetween to provide a substantially solid (in cross section) steerable and torquable guide wire.

6. A guide wire according to claim 5 which further includes a coil wire (52) disposed about the distal segment.
